# NEUE EUROPÄISCHE PATENTSCHRIFT

(11) **EP 1 694 230 B2**
(45) Veröffentlichungstag und Bekanntmachung des Hinweises auf die Entscheidung über den Einspruch: **06.07.2016**
(45) Hinweis auf die Patenterteilung: 17.10.2007
(21) Anmeldenummer: 03776746.4
(22) Anmeldetag: 19.12.2003
(51) Int. Cl.: A61B 17/72

(54) **MARKNAGEL**
INTRAMEDULLARY NAIL
SONDE A MOELLE EPINIERE

(43) Veröffentlichungstag der Anmeldung: 30.08.2006
(73) Patentinhaber: Synthes GmbH, 4436 Oberdorf (CH)
(72) Erfinder: SCHLIENGER, André, CH-4144 Arlesheim (CH); BUETTLER, Markus, CH-4117 Mümliswil (CH); SENN, Peter, CH-4437 Waldenburg (CH)
(74) Vertreter: Lusuardi, Werther
(86) Internationale Anmeldenummer: PCT/CH2003/000834
(87) Internationale Veröffentlichungsnummer: WO 2005/058174

(56) Entgegenhaltungen:
- EP-A1- 1 260 188
- EP-A2- 0 565 216
- CH-A5- 687 229
- US-A- 4 776 330
- US-A1- 2002 099 379
- US-A1- 2002 183 750
- US-A1- 2003 069 581
- US-A1- 2003 135 211
- US-A1- 2003 135 212

## Beschreibung

Die Erfindung bezieht sich auf einen intramedullären Markangel gemäss dem Oberbegriff des Patentanspruchs 1.

Aus der FR 1.031.128 CARRIERI ist ein Marknagel mit einer Vielzahl von Querbohrungen, insbesondere auch in seiner distalen Hälfte, bekannt. Nachteilig bei diesem bekannte Marknagel ist der Umstand, dass sämtliche Querbohrungen den gleichen Abstand und die gleiche Richtung aufweisen. Dadurch wirkt eine zwischen zwei anderen Querbohrungen liegende mittlere Querbohrung als Rotationszentrum, für die Knochenfragmentverbindung zum Marknagel, was unerwünscht ist.

Hier will die Erfindung Abhilfe schaffen. Der Erfindung liegt die Aufgabe zugrunde, einen Marknagel mit mehreren distalen Verriegelungsbohrungen zu schaffen, welcher nach erfolgter Plazierung der distalen Verriegelungsschrauben, das mit diesen Verriegelungsschrauben fixiert Knochenfragment bezüglich Abkippung relativ zur Nagelachse optimaler stabilisiert als dies mit herkömmlichen Marknägeln möglich ist.

Die Erfindung löst die gestellte Aufgabe mit einem Marknagel, welcher die Merkmale des Anspruchs 1 aufweist.

Die durch die Erfindung erreichten Vorteile sind im wesentlichen darin zu sehen, dass dank dem erfindungsgemässen Marknagel mit den in derdistalen Hälfte vorhandenen Verriegelungsoptionen die Freiheit einer Abkippung für das mit der Verriegelungsschraube fixiert Knochenfragment bezüglich der Nagelachse signifikant reduziert wird.

Bei einer besonderen Ausführungsform der Erfindung weist die Bohrungsachse mindestens einer der in der distalen Hälfte liegenden N Querbohrungen einen Abstand y_{N} > 0 von der Zentralachse auf. Im speziellen kann die Bohrungsachse der mittleren Querbohrung einen Abstand y₁ > 0 und die Bohrungsachse der proximalen Querbohrung einen Abstand y₂ > 0 von der Zentralachse (4) aufweisen.

Bei einer weiteren Ausführungsform liegt der Abstand "a" im Bereich von 0,6 (a+b) bis 0,9 (a+b) und vorzugsweise im Bereich von 0,7 (ä+b) bis 0,8 (a+b). Der Abstand "b" kann im Bereich ,von 0,6 (a+b) bis 0,9 (a+b) und vorzugsweise im Bereich von 0,7 (a+b) bis 0,8 (a+b) liegen.

Bei einer weiteren Ausführungsform durchdringen sich - mindesten teilweise - zwei oder mehrere der Querbohrungen (5,6,7) in der distalen Hälfte (3) gegenseitig.

Bei einer weiteren Ausführungsform gelten bei einer Anzahl N > 3 von Querbohrungen die Merkmale A, B,C,D des Anspruchs 1 für jede beliebige Unterkombination von drei Querbohrungen:

Typischerweise liegt die Summe L = (a+b) im Bereich von 20 mm bis 35 mm, vorzugsweise im Bereich von 24 mm bis 30 mm.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht des Marknagels;
Fig. 2 eine Seitenansicht des Marknagels nach Fig. 1;
Fig. 3 eine gegenüber Fig. 2 um 90° verdrehte Seitenansicht des Marknagels nach Fig. 1;
Fig. 4 eine perspektivische Ansicht eines modifizierten Marknagels;
Fig. 5 eine Seitenansicht des Marknagels nach Fig. 4; und
Fig. 6 eine gegenüber Fig. 5 um 90° verdrehte Seitenansicht des Marknagels nach Fig. 4.

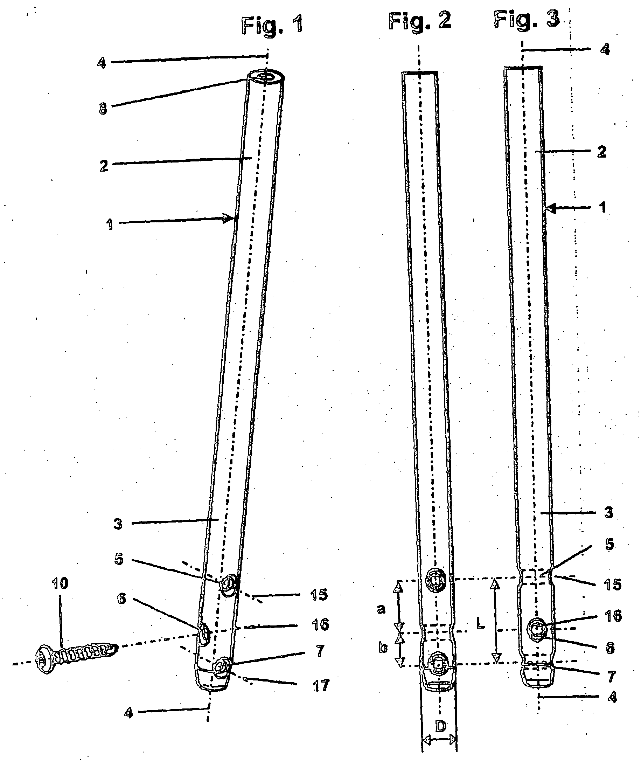

Die in den Fig. 1 - 3 dargestellte erste Ausführungsform eines Marknagels 1 besitzt eine proximale Hälfte, eine zur Einführung in den Markkanal geeignete, distale Hälfte 3 und eine Zentralachse 4 auf. Der Marknagel 1 weist zudem eine durchgehende Kannulierung 8 auf. Die distale Hälfte 3 weist drei, zur Aufnahme von Verriegelungsschrauben 10 geeignete Querbohrungen 5,6,7 auf. Die proximal gelegene Querbohrung 5 besitzt eine Bohrungsachse 15, die mittlere Querbohrung 6 eine Bohrungsachse 16 und die distale Querbohrung 7 eine Bohrungsachse 17. Die Bohrungsachse 16, der zwischen der proximalen Querbohrung 5 und der distalen Querbohrung 7 verlaufenden mittleren Querbohrung 6 weist einen kürzesten Abstand "a" = 16 mm zur Bohrungsachse 15 derproximalen Querbohrung 5 auf, sowie einen kürzesten Abstand "b" = 11 mm zur Bohrungsachse 17 der distalen Querbohrung 7 auf. Im weiteren gilt die Bedingung a ≠ b (a ungleich b). Die Summe L = (a+b) beträgt 27 mm. Der Durchmesser des Marknagels 1 beträgt D = 10 mm.

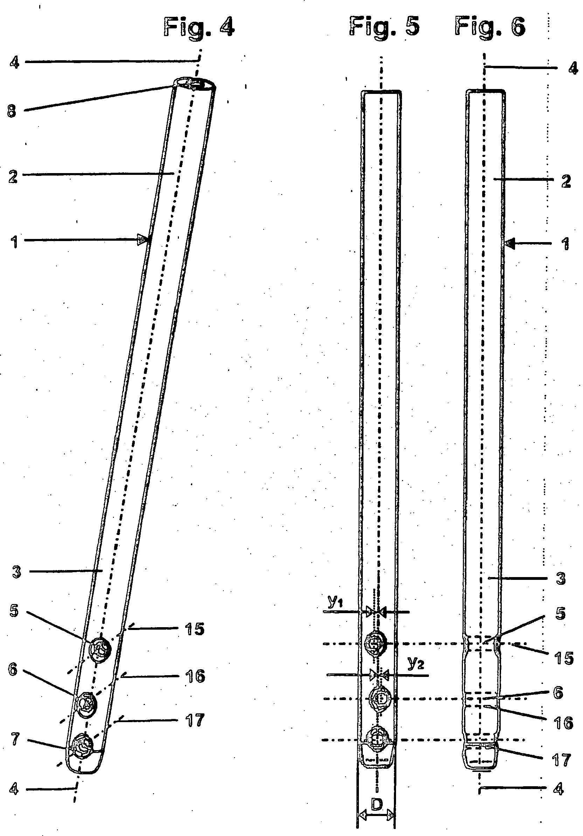

Die in den Fig. 4 - 6 dargestellte zweite Ausführungsform eines Marknagels 1 besitzt im wesentlichen die gleichen Merkmale wie diejenige der ersten Ausführungsform, jedoch mit folgenden zusätzlichen oder abweichenden Parametern:

Die Bohrungsachse 15 der Querbohrung 5 weist einen Abstand y₁ = 0,5 mm zur Zentralachse 4 auf. Auch die Bohrungsachse 16 der Querbohrung 6 weist einen Abstand y₂= 0,5 mm zur Zentralachse 4 auf, jedoch auf die entgegengesetzte Seite.

## Patentansprüche

1. Intramedullärer Marknagel (1) mit einer proximalen Hälfte (2), einer zur Einführung in den Markkanal geeigneten, distalen Hälfte (3) und einer Zentralachse (4), wobei die distale Hälfte (3) mindestens drei, zur Aufnahme von Verriegelungsschrauben (10) geeignete Querbohrungen (5,6,7) aufweist, wobei
A) die distale Hälfte (3) entlang der Zentralachse (4) mindesten eine proximale Querbohrung (5) mit der Bohrungsachse (15), eine mittlere Querbohrung (6) mit der Bohrungsachse (16) und eine distale Querbohrung (7) mit der Bohrungsachse (17) aufweist;
B) die Bohrungsachse (16) der zwischen der proximalen (5) und der distalen (7) Querbohrung verlaufenden mittleren Querbohrung (6) einen kürzesten Abstand "a" zur Bohrungsachse (15) der proximalen Querbohrung (5) aufweist;
C) die Bohrungsachse (16) der zwischen der proximalen (5) und der distalen (7) Querbohrung verlaufenden mittleren Querbohrung (6) einen kürzesten Abstand "b" zur Bohrungsachse (17) der distalen Querbohrung (7) aufweist; und
D) die Bedingung a ≠ b gilt,
**dadurch gekennzeichnet, dass**
E) die Bohrungsachse (15,16,17) mindestens einer der in der distalen Hälfte (3) liegenden N Querbohrungen (5,6,7) einen Abstand y_{N} > 0 von der Zentralachse (4) aufweist; und
F) die Querbohrungen (5,6,7) den Marknagel (1) vollständig durchdringen und als über den Umfang geschlossene Querbohrungen (5, 6, 7) ausgebildet sind.

2. Marknagel (1) nach Anspruch 1, **dadurch gekennzeichnet, dass** die Bohrungsachse (16) der mittleren Querbohrung (6) einen Abstand y₁ > 0 von der Zentralachse (4) aufweist.

3. Marknagel (1) nach Anspruch 1 oder 2, **dadurch gekennzeichnet, dass die** Bohrungsachse (15) der proximalen Querbohrung (5) einen Abstand y₂ > 0 von der Zentralachse (4) aufweist.

4. Marknagel (1) nach einem der Ansprüche 1 bis 3, **dadurch gekennzeichnet, dass** "a" im Bereich von 0,6 (a+b) bis 0,9 (a+b) liegt.

5. Marknagel (1) nach Anspruch 4, **dadurch gekennzeichnet, dass** "a" im Bereich von 0,7 (a+b) bis 0,8 (a+b) liegt.

6. Marknagel (1) nach einem der Ansprüche 1 bis 5, **dadurch gekennzeichnet, dass** "b" im Bereich von 0,6 (a+b) bis 0,9 (a+b) und vorzugsweise im Bereich von 0,7 (a+b) bis 0,8 (a+b) liegt.

7. Marknagel (1) nach einem der Ansprüche 1 bis 6, **dadurch gekennzeichnet, dass** sich zwei oder mehrere der Querbohrungen (5,6,7) in der distalen Hälfte (3) gegenseitig - mindestens teilweise - durchdringen.

8. Marknagel nach einem der Ansprüche 1 7, **dadurch gekennzeichnet, dass** bei einer Anzahl N > 3 von Querbohrungen (5,6,7) die Merkmale A,B,C, D des Anspruchs 1 für jede beliebige Unterkombination von drei Querbohrungen gilt.

9. Marknagel nach einem der Ansprüche 1 - 8, **dadurch gekennzeichnet, dass** die Summe L= (a+b) im Bereich von 20 mm bis 35 mm, vorzugsweise im Bereich von 24 mm bis 30 mm liegt.

10. Marknagel nach einem der Ansprüche 1 - 9, **dadurch gekennzeichnet, dass** er eine zur Zentralachse (4) koaxiale, durchgehende Kannulierung (8) aufweist.

## Claims

1. An intramedullary nail having a proximal half (2), a distal half (3) suitable for being inserted into the medullary canal, and a central axis (4), the distal half (3) being provided with at least three transverse bores (5, 6, 7) suitable for receiving locking screws (10), whereby
A) the distal half (3) is provided along the central axis (4) with at least a proximal transverse bore (5) having a bore axis (15), a central transverse bore (6) having a bore axis (16), and a distal transverse bore (7) having a bore axis (17);
B) the bore axis (16) of the central transverse bore (6), which extends between the proximal transverse bore (5) and the distal transverse bore (7), is spaced at a shortest distance "a" from the bore axis (15) of the proximal transverse bore (5);
C) the bore axis (16) of the central transverse bore (6), which extends between the proximal transverse bore (5) and the distal transverse bore (7), is spaced at a shortest distance "b" from the bore axis (17) of the distal transverse bore (7); and
D) the condition a ≠ b is applicable,
**characterised in that**
E) the bore axis (15, 16, 17) of at least one of the N transverse bores (5, 6, 7) located in the distal half (3) is spaced at a distance y_{N} > 0 from the central axis (4); and
F) the transverse bores (5, 6, 7) completely penetrate the nail (1) and are formed as circumferentially closed transverse bores (5, 6, 7).

2. The intramedullary nail (1) as claimed in claim 1, **characterised in that** the bore axis (16) of the central transverse bore (6) is spaced at a distance y₁ > 0 from the central axis (4).

3. The intramedullary nail (1) as claimed in claim 1 or 2, **characterised in that** the bore axis (15) of the proximal transverse bore (5) is spaced at a distance y₂ > 0 from the central axis (4).

4. The intramedullary nail (1) as claimed in one of the claims 1 to 3, **characterised in that** "a" is in the range of between 0.6 (a+b) and 0.9 (a+b).

5. The intramedullary nail (1) as claimed in claim 4, **characterised in that** "a" is in the range of between 0.7 (a+b) and 0.8 (a+b).

6. The intramedullary nail (1) as claimed in one of the claims 1 to 5, **characterised in that** "b" is in the range of between 0.6 (a+b) and 0.9 (a+b), and preferably in the range of between 0.7 (a+b) and 0.8 (a+b).

7. The intramedullary nail (1) as claimed in one of the claims 1 to 6, **characterised in that** two or more of the transverse bores (5, 6, 7) provided in the distal half (3) intersect one another at least partially.

8. The intramedullary nail (1) as claimed in one of the claims 1 - 7, **characterised in that** with nails displaying a number N > 3 of transverse bores (5, 6, 7), the features A, B, C, D of claim 1 are true of any subcombination of three transverse bores.

9. The intramedullary nail (1) as claimed in one of the claims 1 - 8, **characterised in that** the sum L = (a+b) is in the range of between 20 mm and 35 mm, preferably in the range of between 24 mm and 30 mm.

10. The intramedullary nail (1) as claimed in one of the claims 1 - 9, **characterised in that** it is provided with a cannulation (8) extending coaxially to the central axis (4) over its entire length.

## Revendications

1. Clou intramédullaire (1) comprenant une moitié proximale (2), une moitié distale (3) appropriée pour être introduite dans le canal médullaire (3) et un axe central (4), dans lequel la moitié distale (3) présente au moins trois alésages transversaux (5, 6, 7) appropriés à loger une vis de verrouillage (10), dans lequel
A) la moitié distale (3) présente, le long de l'axe central (4), au moins un alésage transversal proximal (5) présentant l'axe d'alésage (15), un alésage transversal central (6) présentant l'axe d'alésage (16) et un alésage transversal distal (7) présentant l'axe d'alésage (17) ;
B) l'axe d'alésage (16) de l'alésage transversal central (6) s'étendant entre l'alésage transversal proximal (5) et l'alésage transversal distal (7) présente une distance la plus courte "a" par rapport à l'axe d'alésage (15) de l'alésage transversal proximal (5) ;
B) l'axe d'alésage (16) de l'alésage transversal central (6) s'étendant entre l'alésage transversal proximal (5) et l'alésage transversal distal (7) présente une distance la plus courte "b" par rapport à l'axe d'alésage (17) de l'alésage transversal distal (7) ; et
D) la condition a ≠ b s'applique,
**caractérisé en ce que**
E) l'axe d'alésage (15, 16, 17) d'au moins un des N alésages transversaux (5, 6, 7) situés dans la moitié distale (3) présente une distance y_{N} > 0 par rapport à l'axe central (4) ; et
F) les alésages transversaux (5, 6, 7) pénètrent entièrement le clou intramédullaire (1) et sont réalisés en forme d'alésages transversaux (5, 6, 7) fermés sur la circonférence.

2. Clou intramédullaire (1) selon la revendication 1, **caractérisé en ce que** l'axe d'alésage (16) de l'alésage transversal central (6) présente une distance y₁ > 0 par rapport à l'axe central (4).

3. Clou intramédullaire (1) selon la revendication 1 ou 2, **caractérisé en ce que** l'axe d'alésage (15) de l'alésage transversal proximal (5) présente une distance y₂ > 0 par rapport à l'axe central (4).

4. Clou intramédullaire (1) selon l'une quelconque des revendications 1 à 3, **caractérisé en ce que** "a" est dans la gamme de 0,6 (a + b) à 0,9 (a + b).

5. Clou intramédullaire (1) selon la revendication 4, **caractérisé en ce que** "a" est dans la gamme de 0,7 (a + b) à 0,8 (a + b).

6. Clou intramédullaire (1) selon l'une quelconque des revendications 1 à 5, **caractérisé en ce que** "b" est dans la gamme de 0,6 (a + b) à 0,9 (a + b) et de préférence dans la gamme de 0,7 (a + b) à 0,8 (a + b).

7. Clou intramédullaire (1) selon l'une quelconque des revendications 1 à 6, **caractérisé en ce que** deux ou plus des alésages transversaux (5, 8, 7) dans la moitié distale (3) se traversent les uns les autres, au moins en partie.

8. Clou intramédullaire selon l'une quelconque des revendications 1 à 7, **caractérisé en ce que**, pour un nombre d'alésages transversaux (5, 6, 7) N > 3, les caractéristiques A, B, C, D de la revendication 1 sont valables pour chaque sous-combinaison de trois alésages transversaux.

9. Clou intramédullaire selon l'une quelconque des revendications 1 à 8, **caractérisé en ce que** la somme L = (a + b) est dans la gamme de 20 mm à 35 mm, de préférence dans la gamme de 24 mm à 30 mm.

10. Clou intramédullaire selon l'une quelconque des revendications 1 à 9, **caractérisé en ce qu'**il présente un canal traversant (8) coaxial à l'axe central (4).
